# EUROPÄISCHE PATENTSCHRIFT

(11) **EP 3 786 975 B1**
(45) Veröffentlichungstag und Bekanntmachung des Hinweises auf die Patenterteilung: **27.10.2021**
(21) Anmeldenummer: 20190369.7
(22) Anmeldetag: 10.08.2020
(51) Int. Cl.: G16H 40/63

(54) **VERFAHREN ZUR SICHEREN STEUERUNG UND SYSTEM**
METHOD FOR SECURE CONTROL AND SYSTEM
PROCÉDÉ DE COMMANDE SÉCURISÉE ET SYSTÈME

(30) Priorität: 26.08.2019 DE 102019122868
(43) Veröffentlichungstag der Anmeldung: 03.03.2021
(73) Patentinhaber: Karl Storz SE & Co. KG, 78532 Baden-Württemberg Tuttlingen (DE)
(72) Erfinder: Brüderle, Klaus, 78532 Tuttlingen Baden-Württermberg (DE)
(74) Vertreter: Witte, Weller & Partner Patentanwälte mbB

(56) Entgegenhaltungen:
- DE-A1-102013 100 428
- US-A1- 2017 140 169

## Beschreibung

Die vorliegende Erfindung betrifft ein Verfahren zur sicheren Steuerung eines medizinischen Geräts über eine Eingabeeinheit unter Verwendung einer Verifikationseinheit, wobei die Eingabeeinheit mit der Verifikationseinheit über einen Kommunikationskanal kommuniziert. Die Erfindung betrifft ferner ein System mit mindestens einem Prozessor und mindestens einem Speicher, wobei der mindestens eine Speicher Anweisungen enthält, um ein Verfahren zur sicheren Steuerung auszuführen.

Medizinische Geräte in einem Operationssaal weisen in der Regel eine Bedieneinheit auf, die an dem medizinischen Gerät angeordnet ist oder fest mit dem medizinischen Gerät verbunden ist. Auf diese Weise ist sichergestellt, dass die Zuordnung zwischen der Bedieneinheit und dem medizinischen Gerät eindeutig gegeben ist und Steuersignale von der Bedieneinheit auch tatsächlich nur das entsprechende medizinische Gerät erreichen.

Heutige Operationssäle verfügen über eine umfangreiche Netzwerkstruktur, sei diese kabelgebunden oder kabellos, die es ermöglicht, medizinische Geräte mit einer Bedieneinheit zu steuern, die über das Netzwerk mit dem medizinischen Gerät verbunden ist. Dies ermöglicht eine große Flexibilität bei der Anordnung der Bedieneinheit und es kann ermöglicht werden, ein medizinisches Gerät mittels mehrerer Bedieneinheiten zu steuern.

Die Netzwerkstruktur führt jedoch dazu, dass die im Bereich der Medizinprodukte geforderten Sicherheitsanforderungen nicht mehr ohne Weiteres gewährleistet sind. Um dennoch die erforderliche Sicherheit zu gewährleisten, wird einer Bedieneinheit ein separater Freigabeschalter zugeordnet, der mit dem medizinischen Gerät über einen logisch und physikalisch getrennten Signalpfad kommuniziert. Wenn das medizinische Gerät von der Bedieneinheit einen Steuerbefehl erhält, wird der Steuerbefehl nur dann ausgeführt, wenn gleichzeitig der Freigabeschalter dieser Bedieneinheit betätigt wird. So kann sichergestellt werden, dass ein Befehl, der am medizinischen Gerät ankommt, aber nicht von der entsprechenden Bedieneinheit gesendet wurde, vom medizinischen Gerät nicht ausgeführt wird.

Eine solche Lösung ist zuverlässig und kann prinzipiell mit beliebigen medizinischen Geräten und Bedieneinheit eingesetzt werden. Um die notwendige Sicherheit zu erzielen, insbesondere die Sicherheitsklasse C nach der Norm IEC 62304, ist es jedoch erforderlich, den Freigabeschalter jeweils über den genannten, vom Netzwerk separaten Signalpfad mit dem medizinischen Gerät zu verbinden. Ein solches System ist beispielsweise von DE102013100428 A1 bekannt.

Es ist eine Aufgabe der vorliegenden Erfindung, ein Verfahren zur sicheren Steuerung sowie ein entsprechendes System aufzuzeigen, bei denen auch ohne einen separaten Freigabeschalter die für medizinische Geräte erforderliche Sicherheit gewährleistet werden kann.

Gemäß einem ersten Aspekt wird die Aufgabe gelöst durch ein Verfahren zur sicheren Steuerung eines medizinischen Geräts über eine Eingabeeinheit unter Verwendung einer Verifikationseinheit, wobei die Eingabeeinheit mit der Verifikationseinheit über einen Kommunikationskanal kommuniziert, das Verfahren mit den Schritten:
- erstes Erfassen, durch die Eingabeeinheit, einer ersten Betätigung an einem ersten Betätigungspunkt innerhalb eines Freigabebereichs auf der Eingabeeinheit, wobei die erste Betätigung eine Berührung und ein fortwährendes Halten der Berührung ist, wobei das Verfahren mit dem ersten Erfassen fortgesetzt wird, falls die Berührung beendet wird,
- erstes Senden, von der Eingabeeinheit an die Verifikationseinheit, einer Anforderung einer eindeutigen Kontextinformation, die eine eindeutige Zuordnung zwischen der Betätigung auf der Eingabeeinheit und der Verifikationseinheit herstellt,
- Erzeugen, durch die Verifikationseinheit, der Kontextinformation,
- zweites Senden, durch die Verifikationseinheit an die Eingabeeinheit, der Kontextinformation,
- zweites Erfassen, durch die Eingabeeinheit, einer zweiten Betätigung, wobei die zweite Betätigung eine Bewegung vom ersten Betätigungspunkt zu einem zweiten Betätigungspunkt innerhalb eines Steuerbereichs auf der Eingabeeinheit bei fortwährendem Halten der Berührung ist, wobei das Verfahren mit dem ersten Erfassen fortgesetzt wird, falls die Berührung beendet wird, und
- Ansteuern des medizinischen Geräts mit den folgenden sich wiederholenden Schritten, wobei das Verfahren mit dem ersten Erfassen fortgesetzt wird, falls die Berührung beendet wird:
- drittes Erfassen, durch die Eingabeeinheit, einer dritten Betätigung, wobei die dritte Betätigung eine Verlagerung innerhalb des Steuerbereichs bei fortwährendem Halten der Berührung ist,
- drittes Senden, von der Eingabeeinheit an die Verifikationseinheit, einer Steuernachricht, die einen Steuerbefehl, eine Identifikationsinformation und eine Verlagerungsinformation aufweist, wobei der Steuerbefehl dem Steuerbereich zugeordnet ist, die Identifikationsinformation die Kontextinformation oder eine aus der Kontextinformation abgeleitete Information aufweist und die Verlagerungsinformation Informationen aufweist, in der Länge und Richtung der Verlagerung enthalten sind oder daraus ermittelt werden können,
- erstes Überprüfen, durch die Verifikationseinheit, ob die Verlagerungsinformationen von zumindest einer Teilmenge der erfolgten dritten Betätigungen zeigt, dass die Verlagerung auf der Eingabeeinheit einer vorgegebenen Form folgt,
- zweites Überprüfen, durch die Verifikationseinheit, ob die Identifikationsinformation mit der Kontextinformation korrespondiert, und
- viertes Senden, durch die Verifikationseinheit an das medizinische Gerät, des Steuerbefehls oder eines aus dem Steuerbefehl abgeleiteten Befehls, wenn das erste Überprüfen und das zweite Überprüfen positiv abgeschlossen wurden.

Eine der Besonderheiten dieses Verfahrens ist, dass zunächst in einem ersten Schritt, bevor überhaupt ein Steuerbefehl erzeugt wird, eine eindeutige Verbindung zwischen der Eingabeeinheit und dem medizinischen Gerät hergestellt wird. Dies erfolgt über die genannte Kontextinformation. Diese Kontextinformation, die von der Verifikationseinheit erzeugt wird, gibt der Verifikationseinheit die Möglichkeit, im weiteren Verlauf des Verfahrens, eine Steuernachricht, und damit einen Steuerbefehl, eindeutig der Eingabeeinheit zuzuordnen. Ferner kann sich die Eingabeeinheit gegenüber der Verifikationseinheit mittels der Identifikationsinformation identifizieren, die entweder diese Kontextinformation aufweist oder eine abgeleitete Information aufweist, die eindeutig einen Rückschluss auf die Kontextinformation ermöglicht.

Der Begriff "eindeutig" wie er in dieser Anmeldung verwendet wird umfasst einerseits die mathematisch exakte Eindeutigkeit. Andererseits soll im Rahmen der Offenbarung auch eine solche Information als eindeutig verstanden werden, die sich nicht erraten lässt oder nur mit einer impraktikablen Anzahl an Versuchen durch Probieren ermittelt werden kann. So könnte die Identifikationsinformation beispielsweise eine Prüfsumme der Kontextinformation aufweisen und nicht die Kontextinformation selbst. Verwendet man beispielsweise ein Prüfsummenverfahren, welches über eine Milliarde, eine Billion oder mehr Möglichkeiten der Prüfsumme bietet, so soll die Zuordnung der Identifikationsinformation zur Kontextinformation immer noch als eindeutig verstanden werden, da es nicht praktikabel ist, durch Zufall die korrekte Prüfsumme zu erraten ohne die Kontextinformation tatsächlich zu kennen.

Somit wird durch das Erzeugen der Kontextinformation durch die Verifikationseinheit, das Senden der Kontextinformation an die Eingabeeinheit und das spätere Senden der Identifikationsinformation von der Eingabeeinheit an die Verifikationseinheit sichergestellt, dass nur diese eindeutige Zuordnung zum Steuern des medizinischen Geräts zulässig ist.

Ferner ist sichergestellt, dass die eindeutige Zuordnung nur aktiv durch den Benutzer hergestellt werden kann. Es erfordert nämlich die Eingabe des Benutzers an der Eingabeeinheit in einem speziellen Bereich der Eingabeeinheit, nämlich dem Freigabebereich. Nur dann sendet die Eingabeeinheit überhaupt die Anforderung für die Kontextinformation.

Eine versehentliche oder falsche Bedienung der Eingabeeinheit wird danach durch das Erfordernis der zweiten Betätigung verhindert. Diese zweite Betätigung erfordert es, dass der Benutzer unter kontinuierlicher Berührung beispielsweise seinen Finger vom ersten Betätigungspunkt im Freigabebereich zu einem zweiten Betätigungspunkt innerhalb des Steuerbereichs verlagert. Findet diese zweite Betätigung nicht statt, wartet das Verfahren weiterhin auf die zweite Betätigung, zumindest für eine vorgegebene Zeit.

Endet die Berührung, selbst wenn sie nur kurzzeitig unterbrochen wird, springt das Verfahren bevorzugt in einen Ausgangszustand zurück. Die gezielte Verlagerung des Betätigungspunkts auf der Eingabeeinheit vom Freigabebereich zum Steuerbereich kann nur mit geringer Wahrscheinlichkeit zufällig nachgebildet werden. Somit wird sichergestellt, dass der Benutzer auch tatsächlich das medizinische Gerät über die Eingabeeinheit steuern will.

Auch die tatsächliche Steuerung des medizinischen Geräts über die Eingabeeinheit stellt sicher, dass die Steuerung aktiv durch den Benutzer gewollt ist und zweifelsfrei und ausschließlich über die eindeutig zugeordnete Eingabeeinheit erfolgt. Dies erfolgt anhand zweier unabhängiger Überprüfungen, die beide positiv abgeschlossen werden müssen, damit das medizinische Gerät tatsächlich angesteuert wird.

Zum einen wird überprüft, ob eine Betätigung des Benutzers innerhalb des Steuerbereichs der Eingabeeinheit einer vorgegebenen Form folgt. Bevorzugt erfordert ein Folgen der vorgegebenen Form eine möglichst häufige Änderung hinsichtlich der Länge und/oder der Richtung der einzelnen Stücke der Verlagerung, die erforderlich sind, um der Form zu folgen.

Zwar ist es grundsätzlich möglich, auch eine sehr einfache Form zu verwenden, beispielsweise eine Gerade, doch kann es dabei schwierig sein, eine tatsächliche gleichmäßige Bewegung, d.h. Verlagerungen, die stets dieselbe Länge und Richtung haben, von einer Situation zu unterscheiden, wenn aufgrund eines Systemfehlers immer wieder ein eingefrorener Wert von Länge und Richtung gesendet wird. Wird die Form hingegen mit vielen und ggf. fortlaufenden Richtungsänderungen erzeugt, so sind eingefrorene Werte von Länge und Richtung der Verlagerung schnell zu erkennen.

Der Begriff des aus dem Steuerbefehl abgeleiteten Befehls soll dahingehend verstanden werden, dass es nicht erforderlich ist, dass die Verifikationseinheit dem von der Eingabeeinheit gesendeten Steuerbefehl exakt entspricht. So ist es auch möglich, dass die Verifikationseinheit einen Steuerbefehl in einem bestimmten Format oder mit einer bestimmten Information erhält, daraus aber ein Befehl abgeleitet werden muss, der dem Eingabeformat oder der Eingabeinformation entspricht, die das medizinische Gerät erwartet.

Es sei dabei darauf hingewiesen, dass die Eingabeeinheit lediglich die Verlagerungsinformation sendet, nicht jedoch ausschließlich selbst prüft, ob die Verlagerung innerhalb des Steuerbereichs durch den Benutzer der vorgegebenen Form folgt. Würde die Eingabeeinheit nämlich beispielsweise einfach anhand eines Bits mitteilen, ob die Verlagerung gemäß der vorgegebenen Form erfolgt, so könnte das Kippen eines Bits auf der Übertragungsstrecke bei der Verifikationseinheit zu einem fehlerhaften Rückschluss führen. Stattdessen wertet die Verifikationseinheit die Verlagerungsinformation aus, um die Form der Verlagerung auf der Eingabeeinheit zu überprüfen.

Grundsätzlich können alle Verlagerungsinformationen von allen erfolgten dritten Betätigungen berücksichtigt werden. Es ist in aller Regel aber ausreichend, lediglich eine Teilmenge der erfolgten dritten Betätigungen auszuwerten, insbesondere wenn die dritten Betätigungen in einem sehr engen zeitlichen Raster erfolgen. Ferner kann es hilfreich sein, bestimmte Verlagerungsinformationen außer Acht zu lassen, beispielsweise wenn der Benutzer die Betätigung für eine kurze Zeit ruhen lässt, z.B. für einen Zeitraum von bis zu einigen hundert Millisekunden.

Die Verlagerungsinformation kann auf vielfältige Weise ausgestaltet sein. So kann die Verlagerungsinformation beispielsweise unmittelbar die Länge und die Richtung der dritten Betätigung enthalten. Die Verlagerungsinformation kann aber auch die Koordinaten des aktuellen Berührungspunkts übermitteln. So kann in Kenntnis des letzten Berührungspunkts auch Länge und die Richtung der dritten Betätigung ermittelt werden. Zudem kann auch eine Startposition der Verlagerung übermittelt werden, um weitere Überprüfungen und Plausibilitätsbetrachtungen durchzuführen.

Ferner wird auch die zweite Überprüfung durchgeführt, bei der geprüft wird, ob die Identifikationsinformation mit der Kontextinformation korrespondiert. Ziel dieser Überprüfung ist es zu ermitteln, ob der Sender der Identifikationsinformation im Besitz der Kontextinformation ist. Wenn die Identifikationsinformation die Kontextinformation aufweist oder gleich der Kontextinformation ist, ist diese Feststellung besonders einfach zu treffen. Es kann aber durchaus ausreichend sein, dass die Identifikationsinformation nur Teile der Kontextinformation enthält, sofern sichergestellt ist, dass die Kenntnis der Kontextinformation nicht auf eine praktikable Weise vorgetäuscht werden.

Die Identifikationsinformation kann auch Information enthalten, die aus der Kontextinformation abgeleitet ist, wie beispielsweise die oben erläuterte Prüfsumme. Da zumindest periodisch, bevorzugt kontinuierlich, geprüft wird, ob die Identifikationsinformation in der Steuernachricht mit der Kontextinformation korrespondiert, wird fortlaufend sichergestellt, dass Steuerbefehle ausschließlich im Kontext der eindeutigen Zuordnung zwischen der Eingabeeinheit und der Verifikationseinheit akzeptiert werden.

Da ferner sowohl der eindeutige Beginn des Steuervorgangs als auch der fortlaufende Wunsch nach einer aktiven Steuerung durch den Benutzer geprüft werden, ist eine sichere Steuerung gegeben.

Es sei darauf hingewiesen, dass die Betätigung, Berührung, Bewegung und Halten sowohl haptisch, also in physikalischem Kontakt mit der Eingabeeinheit erfolgen können, wie zum Beispiel bei einem Touchscreen, als auch virtuell erfolgen können, wie zum Beispiel in Verbindung mit einer Virtual Reality Brille, wenn der Benutzer aus seiner Perspektive eine virtuelle Oberfläche berührt.

Das Verfahren ist aufgrund der vorgeschlagenen Ausgestaltung so sicher, dass es gemäß der Norm IEC 62304 die Sicherheitsklasse C erfüllen kann. Auch regulatorische Vorgaben aus dem Ausland können erfüllt werden, siehe z.B. "General Principles of Software Validation", abrufbar unter https://www.fda.gov/regulatory-information/search-fda-guidance-documents/general-principles-software-validation. Das heißt unter anderem, dass das Verfahren auch bei höchsten Sicherheitsanforderungen eingesetzt werden kann.

Dadurch ist die Aufgabe vollständig gelöst.

Bei einer vorteilhaften Ausgestaltung werden die jeweils nachfolgenden Schritte des Verfahrens nicht ausgeführt, falls während der Ausführung des Verfahrens die Berührung beendet wird.

Auf diese Weise wird sichergestellt, dass bei einer unterbrochenen Berührung keine Ansteuerung stattfinden kann oder eine begonnene Ansteuerung beendet wird.

Bei einer weiteren vorteilhaften Ausgestaltung wird das Verfahren mit dem ersten Erfassen fortgesetzt, falls während der Ausführung des Verfahrens die Berührung beendet wird.

Auf diese Weise wird sichergestellt, dass das Verfahren bei einer unterbrochenen Berührung in einen definierten Ausgangszustand zurückkehrt und damit keine Ansteuerung stattfinden kann oder eine begonnene Ansteuerung beendet wird.

Bei einer weiteren vorteilhaften Ausgestaltung weist die Verlagerungsinformation einen Befehlsprozessor und einen Verifikationsprozessor auf und stellt mit dem Befehlsprozessor den Steuerbefehl oder den aus dem Steuerbefehl abgeleiteten Befehl für das vierte Senden bereit und gibt mit dem Verifikationsprozessor das vierte Senden frei.

Diese Ausgestaltung ist besonders sicher, da einerseits das Bereitstellen des Steuerbefehls und andererseits das Freigeben des Steuerbefehls, sprich, dass der Steuerbefehl tatsächlich gesendet werden kann, mittels zweier unterschiedlicher physikalischer Bauteile erfolgen. Da der Verifikationsprozessor dann sehr einfach ausgestaltet werden kann und damit auch kostengünstig für eine hohe Sicherheitsklasse ausgelegt werden kann, kann insgesamt die erforderliche Sicherheit der Verifikationseinheit kostengünstig erzielt werden.

Bei einer weiteren vorteilhaften Ausgestaltung weist die Kontextinformation eine Geräteidentifikation der Verifikationseinheit auf.

Auf diese Weise kann während des Verfahrens überprüft werden, ob die von der Eingabeeinheit gesendete Identifikationsinformation tatsächlich für die Verifikationseinheit bestimmt ist. Diese Prüfung kann auch über ein weiteres Element erfolgen, welches sowohl von der Eingabeeinheit als auch von der Verifikationseinheit unabhängig ist. Alternativ oder zusätzlich ist es möglich, dass die Kontextinformation eine Geräteidentifikation des Eingabegeräts aufweist.

Bei einer weiteren vorteilhaften Ausgestaltung weist die Kontextinformation mindestens ein Element auf ausgewählt aus einer Gruppe bestehend aus einer Zeitinformation, einer Sitzungsinformation, einer Sequenzinformation und einer Startinformation.

Diese Ausgestaltung ermöglicht eine oder mehrere weitere Überprüfungen, um sicherzustellen, dass ein Steuerbefehl nur im Kontext der eindeutigen Zuordnung zwischen dem Eingabegerät und der Verifikationseinheit erfolgt. Ferner kann auch die Plausibilität von Steuernachrichten überprüft werden. So kann anhand der Zeitinformation beispielsweise überprüft werden, ob die Steuernachrichten in zeitlich korrekter Reihenfolge eintreffen, ob die Abstände zwischen den Steuernachrichten einen Höchstabstand nicht überschreiten und ob die Laufzeit der Steuernachricht vom Eingabegerät zur Verifikationseinheit einen Höchstwert nicht überschreitet.

Anhand der Sitzungsinformation kann überprüft werden, ob die Steuernachricht im Kontext des zuletzt erfolgten ersten Erfassens steht oder ob hier möglicherweise eine ältere Kontextinformation übermittelt wird, die nicht mehr als gültig verstanden werden soll. Die Sequenzinformation erlaubt es zu überprüfen, ob die Steuernachrichten in der richtigen Reihenfolge eingehen oder ermöglicht es, asynchron eingehende Steuernachrichten in die richtige Reihenfolge zu bringen. Die Startinformation, auch Seedinformation genannt, liefert einen spezifischen Startwert, der nicht auf praktikable Weise zufällig ermittelt werden kann. So kann die Startinformation beispielsweise eine zufällig erzeugte Zahl sein, insbesondere eine große Zahl. Schlägt eine dieser Überprüfungen fehl, kann die empfangene Steuernachricht verworfen werden oder die Steuerung abgebrochen werden und das Verfahren mit dem Schritt des ersten Erfassens fortgesetzt werden.

Bei einer weiteren vorteilhaften Ausgestaltung ist die vorgegebene Form eine Ovalform, eine Ellipsenform oder eine Kreisform.

Bei dieser Ausgestaltung ergeben sich im Formverlauf besonders viele Änderungen der Bewegungsrichtung. Somit kann schnell erkannt werden, falls die Steuernachricht eingefrorene Verlagerungsinformationen enthält, die nur scheinbar auf eine dritte Betätigung hinweisen.

Bei einer weiteren vorteilhaften Ausgestaltung der Erfindung ist der Kommunikationskanal genau ein physikalischer und/oder ein logischer Kanal, und wird insbesondere der Steuerbefehl als genau eine Nachricht gesendet.

Diese Ausgestaltung ist vorteilhaft, da es nicht erforderlich ist, mehrfache physikalische und/oder logische Kanäle zu verwenden. Zudem ist es insbesondere nicht erforderlich, den Steuerbefehl in mehreren Nachrichten zu senden. Dies ermöglicht einen vereinfachten Aufbau für die Verbindung zwischen der Eingabeeinheit und der Verifikationseinheit.

Bei einer weiteren vorteilhaften Ausgestaltung der Erfindung wird das Verfahren mit dem ersten Erfassen fortgesetzt, falls sich die Verlagerungsinformation identisch oder innerhalb eines vorgegebenen Toleranzbereichs wiederholt.

Wie bereits erläutert wird es als vorteilhaft angesehen, wenn das Verfahren so durchgeführt wird, dass ein periodisches oder fortlaufendes Ändern der Steuernachricht und insbesondere der Verlagerungsinformation erwartet werden darf. Insbesondere wird es einem Benutzer nicht gelingen, eine Bewegung so durchzuführen, dass die Verlagerungsinformationen identisch sind oder sich wiederholen. Je nach Auslegung kann außerdem ein Toleranzbereich festgelegt werden, innerhalb dessen eine Verlagerungsinformation immer noch als identisch angesehen werden soll. Dies bedeutet, dass wenn eine identische oder sich wiederholende Verlagerungsinformation erkannt wird, davon auszugehen ist, dass die Betätigung nicht durch einen Benutzer erfolgt, sondern anderweitig erzeugt wird, beispielsweise mittels eines sogenannten Replay. In einem solchen Zustand können bevorzugt Steuernachrichten verworfen werden und/oder die Steuerung abgebrochen werden und/oder das Verfahren mit dem ersten Erfassen fortgesetzt werden.

Gemäß einem zweiten Aspekt wird die Aufgabe gelöst durch ein System mit mindestens einem Prozessor und mindestens einem Speicher, wobei der mindestens eine Speicher Anweisungen enthält, die bewirken, wenn sie von dem mindestens einen Prozessor ausgeführt werden, dass der mindestens eine Prozessor ein zuvor beschriebenes Verfahren ausführt.

Bei einer vorteilhaften Ausgestaltung weist der mindestens eine Prozessor einen Befehlsprozessor und einen Verifikationsprozessor auf, wobei der Verifikationsprozessor für das erste und zweite Überprüfen sowie ein Freigeben des Steuerbefehls oder des aus dem Steuerbefehl abgeleiteten Befehls ausgebildet ist und der Befehlsprozessor für ein Bereitstellen des Steuerbefehls oder des aus dem Steuerbefehl abgeleiteten Befehls ausgebildet ist.

Diese Ausgestaltung ist, wie bereits weiter oben erläutert, besonders sicher, da einerseits das Bereitstellen des Steuerbefehls und andererseits das Freigeben des Steuerbefehls, sprich, dass der Steuerbefehl tatsächlich gesendet werden kann, mittels zweier unterschiedlicher Bauteile erfolgen. Da der Verifikationsprozessor dann sehr einfach ausgestaltet werden kann und damit auch kostengünstig für eine hohe Sicherheitsklasse ausgelegt werden kann, kann insgesamt die erforderliche Sicherheit der Verifikationseinheit kostengünstig erzielt werden.

Bei einer weiteren vorteilhaften Ausgestaltung weist die Eingabeeinheit einen Touchscreen oder eine Gestenerfassungseinheit auf, insbesondere in Verbindung mit einer Virtual Reality-Brille.

Diese Ausgestaltung ermöglicht es, das System sowohl auf Basis einer tatsächlichen Berührung als auch auf Basis einer virtuellen Berührung zu realisieren. Dies ist insbesondere im Hinblick auf die zunehmende Verwendung von Virtual Reality-Brillen interessant, da eine Steuerung dann vom Benutzer auch ohne eine haptische Berührung einer Oberfläche sicher erfolgen kann.

Bei einer weiteren vorteilhaften Ausgestaltung ist die Eingabeeinheit für eine Eingabe mittels Hand und/oder Finger eines Benutzers ausgebildet.

Eine solche Art der Eingabe ist besonders einfach und intuitiv.

Bei einer weiteren vorteilhaften Ausgestaltung ist das medizinische Gerät ein Operationstisch.

Diese Ausgestaltung ist vorteilhaft, da die fortlaufende dritte Betätigung innerhalb des Steuerbereichs intuitiv genau so lange erfolgt, wie der Operationstisch verlagert werden soll. Zudem ist es auf einfache Weise möglich, auf der Eingabeeinheit mindestens zwei Steuerbereiche bereitzustellen, die unterschiedliche Steuerbefehle erzeugen. So können verschiedene Elemente des Operationstischs und/oder verschiedene Bewegungsrichtungen eines Elements des Operationstischs angesteuert werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Figur 1: ein Ausführungsbeispiel eines Verfahrens zur sicheren Steuerung eines medizinischen Geräts über eine Eingabeeinheit;
- Figur 2: ein Ausführungsbeispiel eines Systems mit mindestens einem Prozessor, der ein Verfahren zur sicheren Steuerung eines medizinischen Geräts über eine Eingabeeinheit ausführt; und
- Figur 3: eine Ausführungsform eines Verlaufs von Befehlen und Nachrichten bei einem Verfahren zur sicheren Steuerung eines medizinischen Geräts über eine Eingabeeinheit.

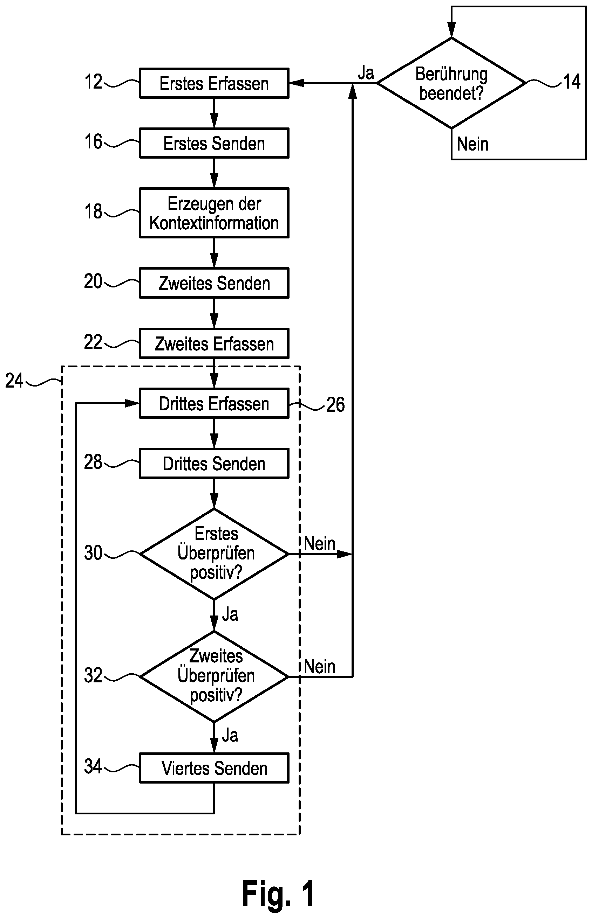

Die nachfolgenden Erläuterungen erfolgen auf Basis einer Zusammenschau der Figur 1 bis 3.

Figur 1 zeigt ein Verfahren zur sicheren Steuerung eines medizinischen Geräts 50 über eine Eingabeeinheit 52. Das Verfahren verwendet eine Verifikationseinheit 54, wobei die Eingabeeinheit 52 mit der Verifikationseinheit 54 über einen Kommunikationskanal 56a, 56b kommuniziert.

Das Verfahren weist ein erstes Erfassen 12, durch die Eingabeeinheit 52, einer ersten Betätigung an einem ersten Betätigungspunkt 58 innerhalb eines Freigabebereichs auf der Eingabeeinheit 52 auf. Dabei ist die erste Betätigung 64a eine Berührung und ein fortwährendes Halten der Berührung.

Es ist hier anhand des Schritts 14 symbolisch dargestellt, dass das Verfahren 10 mit dem ersten Erfassen 12 fortgesetzt wird, falls die Berührung beendet wird. Diese Überprüfung findet bei dieser Ausgestaltung im Hintergrund statt und ist daher nicht einem konkreten Punkt im Ablauf des Verfahrens zugeordnet. Bei anderen Ausgestaltungen wird das Verfahren bei Beendigung der Berührung gestoppt und/oder wird das Verfahren in einen definierten Ausgangszustand gesetzt und/oder wird der Benutzer durch einen Hinweis auf das Beenden der Berührung hingewiesen.

Im Schritt des ersten Sendens 16 sendet die Eingabeeinheit 52 eine Anforderung 106 an die Verifikationseinheit 54, um von dort eine eindeutige Kontextinformation 62 zu erhalten. Die Kontextinformation 62 stellt eine eindeutige Zuordnung zwischen der Betätigung auf der Eingabeeinheit 52 und der Verifikationseinheit 54 her.

Auf die Anforderung 106 durch die Eingabeeinheit 52 erzeugt die Verifikationseinheit 54 im Schritt 18 die Kontextinformation.

Wenn die Kontextinformation 62 erzeugt ist, wird sie durch ein zweites Senden 20 von der Verifikationseinheit 54 an die Eingabeeinheit 52 gesendet.

Es folgt dann ein zweites Erfassen 22, durch die Eingabeeinheit 52, einer zweiten Betätigung 64b. Die zweite Betätigung 64b ist eine Bewegung vom ersten Betätigungspunkt 58 zu einem zweiten Betätigungspunkt 66 innerhalb eines Steuerbereichs 68a auf der Eingabeeinheit 52. Diese Bewegung erfolgt bei fortwährendem Halten der Berührung. Es sei hier daran erinnert, siehe Schritt 14, dass das Verfahren 10 mit dem ersten Erfassen 12 fortgesetzt wird, falls die Berührung beendet wird. Wenn die zweite Betätigung 64a erfasst wurde, wird nun im Schritt 24 das medizinische Gerät 50 mit sich wiederholenden Schritten angesteuert, die nachfolgend näher erläutert werden. Auch hier sei erneut daran erinnert, dass das Verfahren 10 mit dem ersten Erfassen 12 fortgesetzt wird, falls die Berührung beendet wird 14.

Zunächst wird durch die Eingabeeinheit 52 ein drittes Erfassen 26 einer dritten Betätigung 64c durchgeführt. Bei der dritten Betätigung 64c handelt es sich um eine Verlagerung innerhalb des Steuerbereichs 68a bei fortwährendem Halten der Berührung.

Es folgt ein drittes Senden 28, von der Eingabeeinheit 52 an die Verifikationseinheit 54, einer Steuernachricht 70, die einen Steuerbefehl 72, eine Identifikationsinformation 74 und eine Verlagerungsinformation 76 aufweist. Der Steuerbefehl 72 ist dem Steuerbereich 68a zugeordnet. Bei der hier gezeigten Ausführungsform sendet beispielsweise der weitere Steuerbereich 68b einen weiteren Steuerbefehl, der verschieden vom Steuerbefehl 72 ist.

Die Identifikationsinformation 74 weist die Kontextinformation 62 oder eine aus der Kontextinformation 62 abgeleitete Information auf. Dabei kann diese Information beispielsweise durch ein Verkürzen oder ein Erweitern der Kontextinformation 62 erhalten sein oder das Ergebnis einer Berechnung, wie beispielsweise einer Prüfsumme, auf Basis der Kontextinformation 62 sein. Die Verlagerungsinformation 76 weist Informationen auf, in der Länge und Richtung der Verlagerung enthalten sind oder daraus ermittelt werden können. Die zeitliche Auflösung bei der Ermittlung der Verlagerung ist dabei so gewählt, dass aus einer Mehrzahl von Verlagerungsinformationen 76 die Form der vom Benutzer durchgeführten Bewegung auf dem Steuerbereich 68a ermittelt werden kann.

Bei einem ersten Überprüfen 30, durch die Verifikationseinheit 54, wird überprüft, ob die Verlagerungsinformationen, die bisher oder zuletzt erhalten wurden, von zumindest einer Teilmenge der erfolgten dritten Betätigungen zeigt, dass die Verlagerung auf der Eingabeeinheit 52 einer vorgegebenen Form folgt. Ist beispielsweise durch Betrachtung der einzelnen, üblicherweise kurzen Segmente der dritten Betätigung ein Kreisbogen zu erkennen, so kann daraus auf eine Kreisform geschlossen werden.

Bei einem zweiten Überprüfen 32 wird durch die Verifikationseinheit 54 überprüft, ob die Identifikationsinformation 74 mit der Kontextinformation 62 korrespondiert. Dabei soll insbesondere überprüft werden, dass die Identifikationsinformation 74 in Kenntnis der Kontextinformation 62 erfolgt ist und nicht zufällig erzeugt wurde.

Wenn das erste Überprüfen 30 und das zweite Überprüfen 32 positiv abgeschlossen wurden, erfolgt ein viertes Senden 34, durch die Verifikationseinheit 54, an das medizinische Gerät 50, des Steuerbefehls oder eines aus dem Steuerbefehl abgeleiteten Befehls. Das medizinische Gerät 50 wird nun tatsächlich angesteuert. Dies kann beispielsweise bedeuten, dass sich das medizinische Gerät 50 oder ein Teil davon verlagert.

Bei der hier gezeigten Ausführungsform des Verfahrens 10 ist zu erkennen, dass sowohl bei einem negativen Ergebnis des ersten Überprüfens 30, siehe den NEIN-Pfad, als auch bei einem negativen Ergebnis des zweiten Überprüfens 32, siehe den NEIN-Pfad, jeweils eine Rückkehr zum ersten Erfassen 12 erfolgt. Es ist grundsätzlich aber auch möglich, bei einem negativen Ergebnis zunächst zum dritten Erfassen 26 zu springen und erst nach mehreren negativen Prüfungen zurück zum ersten Erfassen 12 zu springen.

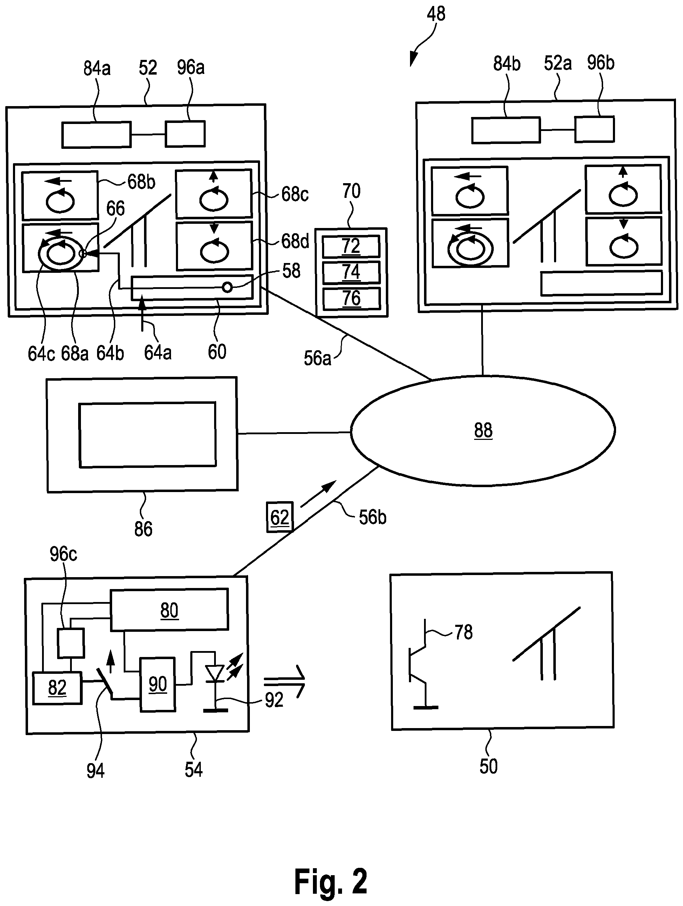

Figur 2 zeigt ein System 48 mit einem Befehlsprozessor 80, einem Verifikationsprozessor 82, einem ersten Eingabegerätprozessor 84a in der Eingabeeinheit 52 und einem zweiten Eingabegerätprozessor 84b in einer weiteren Eingabeeinheit 52a. Ferner ist eine Zentralsteuerung 86 des Systems 48 dargestellt. Die Eingabeeinheit 52 ist über ein Netzwerk 88 mit der Verifikationseinheit 54 verbunden. Hierbei kann es sich beispielsweise um den KARL STORZ® Communication Bus (SCB) handeln.

Das medizinische Gerät 50 ist hier ein Operationstisch und weist einen Empfänger 78 auf. Die Verifikationseinheit 54 weist eine Logikschaltung 90 auf, die nur dann über den Sender 92 einen Befehl sendet, insbesondere mittels Infrarot-Übertragung, wenn sowohl vom Befehlsprozessor 80 ein Steuerbefehl oder ein aus dem Steuerbefehl abgeleiteter Befehl für das vierte Senden 34 bereitgestellt wurde als auch der Verifikationsprozessor 82 das vierte Senden 34 mittels des symbolisch dargestellten Schalters 94 freigibt. Das System 48 weist ferner einen Speicher auf, der sich aus einem ersten Speicher 96a in der Eingabeeinheit 52, einem zweiten Speicher 96b in der weiteren Eingabeeinheit 52a und einem dritten Speicher 96c in der Verifikationseinheit 54 zusammensetzt. Bei der hier gezeigten Ausführungsform enthalten der erste Speicher 96a und der dritte Speicher 96c Anweisungen, die bewirken, wenn sie von dem mindestens einen Prozessor ausgeführt werden, dass der mindestens eine Prozessor ein Verfahren 10 wie zuvor beschrieben ausführt.

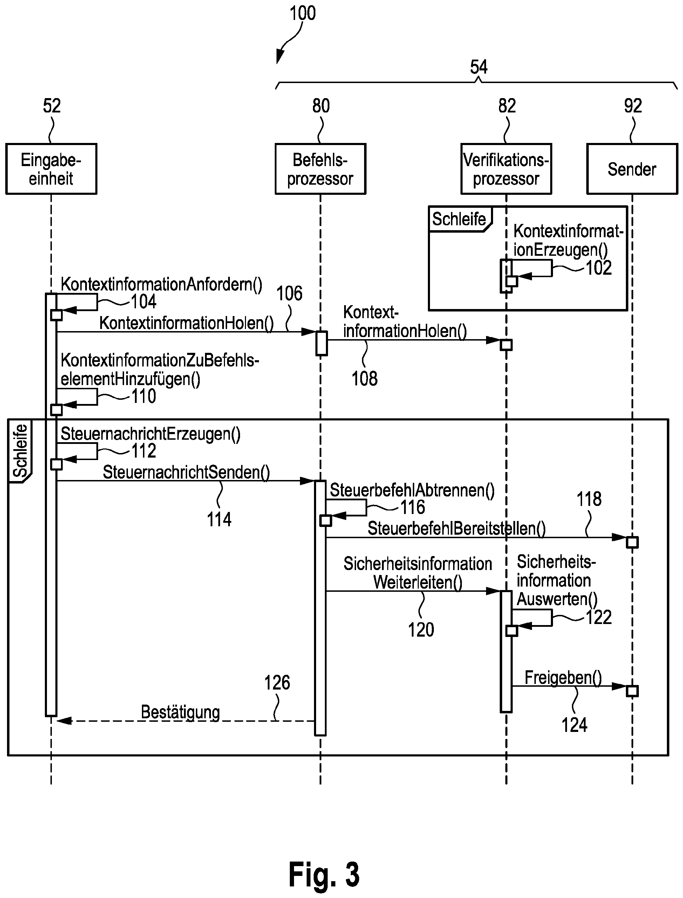

In Figur 3 wird anhand eines Ausführungsbeispiels der Verlauf 100 von Befehlen und Nachrichten an bzw. zwischen den verschiedenen Elementen dargestellt. Zunächst sei auf den Befehl KontextinformationErzeugen() 102 hingewiesen. Dieser wird permanent von dem Verifikationsprozessor 82 ausgeführt, so dass periodisch, z.B. im Abstand von mehreren hundert Millisekunden, insbesondere 200ms bis 800ms, stets eine neue Kontextinformation 62 erzeugt wird.

In der Eingabeeinheit 52 wird zunächst der Befehl KontextinformationAnfordern() 104 ausgeführt. Dann wird die Nachricht KontextinformationHolen() 106 an den Befehlsprozessor 80 gesendet, der hier in Verbindung mit dem Kommunikationskanal 56a, 56b steht. Es ist grundsätzlich aber auch möglich, die Nachricht direkt an den Verifikationsprozessor 82 zu senden, doch würde dies die Komplexität des Verifikationsprozessors 82 erhöhen. Mittels der Nachricht KontextinformationHolen() 108 fordert der Befehlsprozessor 80 die Kontextinformation 62 vom Verifikationsprozessor 82 an, die dann über den Befehlsprozessor 80 zurück zur Eingabeeinheit 52 gesendet wird (nicht gezeigt).

Mittels des Befehls KonfigurationsinformationZuBefehlselementHinzufügen() 110 wird die Kontextinformation 62 mit dem Steuerbereich 68a verknüpft.

Die nachfolgenden Befehle und Nachrichten werden innerhalb einer Schleife durchgeführt bzw. gesendet.

Zunächst wird mittels des Befehls SteuernachrichtErzeugen() 112 die Steuernachricht 70 erzeugt. Mittels der Nachricht SteuernachrichtSenden() 114 wird die Steuernachricht 70 an den Befehlsprozessor 80 gesendet. Dort wird mittels des Befehls SteuerbefehlAbtrennen() 116 der Steuerbefehl 72 von den weiteren Elementen der Steuernachricht 70, also von der Identifikationsinformation 74 und der Verlagerungsinformation 76, getrennt.

Mittels der Nachricht SteuerbefehlBereitstellen() 118 an den Sender 92 wird der Steuerbefehl 72 oder ein aus dem Steuerbefehl 72 abgeleiteter Befehl zum Senden bereitgestellt. Mittels der Nachricht SicherheitsinformationWeiterleiten() werden die Identifikationsinformation 74 und die Verlagerungsinformation 76 an den Verifikationsprozessor 82 weitergeleitet. Mittels des Befehls SicherungsinformationAuswerten() 122 werden das erste Überprüfen 30 und das zweite Überprüfen 32 durchgeführt. Wenn das erste Überprüfen 30 und das zweite Überprüfen 32 positiv abgeschlossen wurden, erfolgt über die Nachricht Freigeben() 124 die Freigabe an den Sender 92, dass der bereitgestellte Steuerbefehl 72 gesendet werden kann. Optional kann über die Nachricht 126 eine Bestätigung vom Befehlsprozessor 82 an die Eingabeeinheit 52 über das erfolgreiche oder nicht-erfolgreiche Ausführen des Steuerbefehls 72 erfolgen.

## Patentansprüche

1. Verfahren (10) zur sicheren Steuerung eines medizinischen Geräts (50) über eine Eingabeeinheit (52) unter Verwendung einer Verifikationseinheit (54), wobei die Eingabeeinheit (52) mit der Verifikationseinheit (54) über einen Kommunikationskanal (56a, 56b) kommuniziert, das Verfahren (10) mit den Schritten:
- erstes Erfassen (12), durch die Eingabeeinheit (52), einer ersten Betätigung (64a) an einem ersten Betätigungspunkt (58) innerhalb eines Freigabebereichs (60) auf der Eingabeeinheit (52), wobei die erste Betätigung (64a) eine Berührung und ein Fortbestehen der Berührung ist,
- erstes Senden (16), von der Eingabeeinheit (52) an die Verifikationseinheit (54), einer Anforderung (106) einer eindeutigen Kontextinformation (62), die eine eindeutige Zuordnung zwischen der Betätigung auf der Eingabeeinheit (52) und der Verifikationseinheit (54) herstellt,
- Erzeugen (18), durch die Verifikationseinheit (54), der Kontextinformation (62),
- zweites Senden (20), durch die Verifikationseinheit (54) an die Eingabeeinheit (52), der Kontextinformation (62),
- zweites Erfassen (22), durch die Eingabeeinheit (52), einer zweiten Betätigung (64b), die bei fortwährendem Halten der Berührung der ersten Betätigung folgt, wobei die zweite Betätigung (64b) eine Bewegung vom ersten Betätigungspunkt (58) zu einem zweiten Betätigungspunkt (66) innerhalb eines Steuerbereichs (68a) auf der Eingabeeinheit (52) bei fortwährendem Halten der Berührung ist, und
- Ansteuern (24) des medizinischen Geräts (50) mit den folgenden sich wiederholenden Schritten:
- drittes Erfassen (26), durch die Eingabeeinheit (52), einer dritten Betätigung (64c), wobei die dritte Betätigung eine Verlagerung innerhalb des Steuerbereichs (68a) bei fortwährendem Halten der Berührung ist,
- drittes Senden (28), von der Eingabeeinheit (52) an die Verifikationseinheit (54), einer Steuernachricht (70), die einen Steuerbefehl (72), eine Identifikationsinformation (74) und eine Verlagerungsinformation (76) aufweist, wobei der Steuerbefehl (72) dem Steuerbereich (68a) zugeordnet ist, die Identifikationsinformation (74) die Kontextinformation (62) oder eine aus der Kontextinformation (62) abgeleitete Information aufweist und die Verlagerungsinformation (76) Informationen aufweist, in der Länge und Richtung der Verlagerung enthalten sind oder daraus ermittelt werden können,
- erstes Überprüfen (30), durch die Verifikationseinheit (54), ob die Verlagerungsinformationen (76) von zumindest einer Teilmenge der erfolgten dritten Betätigungen zeigt, dass die Verlagerung auf der Eingabeeinheit (52) einer vorgegebenen Form folgt,
- zweites Überprüfen (32), durch die Verifikationseinheit (54), ob die Identifikationsinformation (74) mit der Kontextinformation korrespondiert (62), und
- viertes Senden (34), durch die Verifikationseinheit (54) an das medizinische Gerät (50), des Steuerbefehls (72) oder eines aus dem Steuerbefehl (72) abgeleiteten Befehls, wenn das erste Überprüfen (30) und das zweite Überprüfen (32) positiv abgeschlossen wurden.

2. Verfahren nach Anspruch 1, wobei die jeweils nachfolgenden Schritte des Verfahrens nicht ausgeführt werden, falls während der Ausführung des Verfahrens die Berührung beendet wird.

3. Verfahren nach einem der vorhergehenden Ansprüche, wobei das wobei das Verfahren (10) mit dem ersten Erfassen (12) fortgesetzt wird (14), falls während der Ausführung des Verfahrens die Berührung beendet wird.

4. Verfahren nach einem der vorhergehenden Ansprüche, wobei die Verifikationseinheit (54) einen Befehlsprozessor (80) und einen Verifikationsprozessor (82) aufweist und mit dem Befehlsprozessor (80) den Steuerbefehl (72) oder den aus dem Steuerbefehl (72) abgeleiteten Befehl für das vierte Senden (34) bereitstellt und mit dem Verifikationsprozessor (82) das vierte Senden (34) freigibt.

5. Verfahren nach einem der vorhergehenden Ansprüche, wobei die Kontextinformation (62) eine Geräteidentifikation der Verifikationseinheit (54) aufweist.

6. Verfahren nach einem der vorhergehenden Ansprüche, wobei die Kontextinformation (62) mindestens ein Element ausgewählt aus einer Gruppe bestehend aus einer Zeitinformation, einer Sitzungsinformation, einer Sequenzinformation und einer Startinformation aufweist.

7. Verfahren nach einem der vorhergehenden Ansprüche, wobei die vorgegebene Form eine Ovalform, eine Ellipsenform oder eine Kreisform ist.

8. Verfahren nach einem der vorhergehenden Ansprüche, wobei der Kommunikationskanal (56a, 56b) genau ein physikalischer und/oder ein logischer Kanal ist, und insbesondere der Steuerbefehl (72) als genau eine Nachricht gesendet wird.

9. Verfahren nach einem der vorhergehenden Ansprüche, wobei das Verfahren (10) mit dem ersten Erfassen (12) fortgesetzt wird, falls sich die Verlagerungsinformation (76) identisch oder innerhalb eines vorgegebenen Toleranzbereichs wiederholt.

10. System (48) mit einem medizinischen Gerät (50), einer Eingabeeinheit (52), mindestens einem Prozessor und mindestens einem Speicher (96a, 96c), wobei der mindestens eine Speicher (96a, 96c) Anweisungen enthält, die bewirken, wenn sie von dem mindestens einen Prozessor ausgeführt werden, dass der mindestens eine Prozessor ein Verfahren (10) nach einem der vorhergehenden Ansprüche ausführt.

11. System nach Anspruch 10, wobei der mindestens eine Prozessor einen Befehlsprozessor (80) und einen Verifikationsprozessor (82) aufweist, wobei der Verifikationsprozessor (82) für das erste und zweite Überprüfen (30, 32) sowie ein Freigeben (124) des Steuerbefehls (72) oder des aus dem Steuerbefehl (72) abgeleiteten Befehls ausgebildet ist und der Befehlsprozessor (80) für ein Bereitstellen des Steuerbefehls (72) oder des aus dem Steuerbefehl (72) abgeleiteten Befehls ausgebildet ist.

12. System nach Anspruch 10 oder 11, wobei die Eingabeeinheit (52) einen Touchscreen oder eine Gestenerfassungseinheit, insbesondere in Verbindung mit einer Virtual Reality-Brille, aufweist.

13. System nach einem der Ansprüche 10 bis 12, wobei die Eingabeeinheit (52) für eine Eingabe mittels Hand und/oder Finger eines Benutzers ausgebildet ist.

14. System nach einem der Ansprüche 10 bis 13, wobei das medizinische Gerät (50) ein Operationstisch ist.

## Claims

1. A method (10) for safely controlling a medical device (50) via an input unit (52) using a verification unit (54), the input unit (52) communicating with the verification unit (54) via a communication channel (56a, 56b), the method (10) comprising the steps of
- first detecting (12), by the input unit (52), of a first actuation (64a) at a first actuation point (58) within a release area (60) on the input unit (52), wherein the first actuation (64a) is a touch and a maintaining of the touch,
- first sending (16), from the input unit (52) to the verification unit (54), of a request (106) for unambiguous context information (62) that establishes an unambiguous association between the actuation on the input unit (52) and the verification unit (54),
- generating (18), by the verification unit (54), the context information (62),
- second sending (20), by the verification unit (54) to the input unit (52), of the context information (62),
- second detecting (22), by the input unit (52), of a second actuation (64b) that follows the first actuation while continuously maintaining the touch, the second actuation (64b) being a movement from the first actuation point (58) to a second actuation point (66) within a control area (68a) on the input unit (52) while continuously holding the touch, and
- controlling (24) the medical device (50) with the following repetitive steps:
- third detecting (26), by said input unit (52), of a third actuation (64c), said third actuation being a displacement within said control area (68a) while continuously holding said touch,
- third sending (28), from the input unit (52) to the verification unit (54), of a control message (70) comprising a control command (72), identification information (74) and displacement information (76), wherein the control command (72) is associated with the control area (68a), the identification information (74) comprises the context information (62) or an information derived from the context information (62), and the displacement information (76) comprises information in which the length and direction of the displacement is contained or may be determined therefrom,
- first checking (30), by the verification unit (54), whether the displacement information (76) of at least a subset of the third actuations performed shows that the displacement on the input unit (52) follows a predetermined shape,
- second checking (32), by the verification unit (54), whether the identification information (74) corresponds (62) to the context information, and
- fourth sending (34), by the verification unit (54) to the medical device (50), of the control command (72) or a command derived from the control command (72) when the first checking (30) and the second checking (32) have been completed positively.

2. The method of claim 1, whereby the respective subsequent steps of the method are not carried out if the touch is terminated during the execution of the method.

3. The method of any preceding claim, wherein the method (10) continues (14) with the first detecting (12) if the touch is terminated during the execution of the method.

4. The method of any preceding claim, wherein the verification unit (54) comprises an instruction processor (80) and a verification processor (82) and provides with the instruction processor (80) the control instruction (72) or the instruction derived from the control instruction (72) for the fourth sending (34) and enables with the verification processor (82) the fourth sending (34).

5. The method of any preceding claim, wherein the context information (62) comprises a device identification of the verification unit (54).

6. The method of any of the foregoing claims, wherein the context information (62) comprises at least one element selected from a group consisting of time information, session information, sequence information and start information.

7. The method of any of the preceding claims, where the pre-determined shape is an oval shape, an ellipse shape or a circle shape.

8. The method of any preceding claim, wherein the communication channel (56a, 56b) is exactly one physical and/or one logical channel, and in particular the control command (72) is sent as exactly one message.

9. The method of any preceding claim, wherein the method (10) is continued with the first detecting (12) if the displacement information (76) is identical or repeated within a predetermined tolerance range.

10. A system (48) comprising a medical device (50), an input unit, at least one processor and at least one memory (96a, 96c), wherein the at least one memory (96a, 96c) contains instructions which, when executed by the at least one processor, cause the at least one processor to perform the method (10) of one of the foregoing claims.

11. The system of claim 10, wherein the at least one processor comprises an instruction processor (80) and a verification processor (82), wherein the verification processor (82) is configured for the first and second checking (30, 32) and enabling (124) of the control instruction (72) or the instruction derived from the control instruction (72), and the instruction processor (80) is configured for providing the control instruction (72) or the instruction derived from the control instruction (72).

12. The system of claim 10 or 11, wherein the input unit (52) comprises a touch screen or a gesture detection unit, in particular in combination with virtual reality glasses.

13. The system of one of claims 10 to 12, wherein the input unit (52) is configured for input by means of a user's hand and/or finger.

14. The system of any one of claims 10 to 13, wherein the medical device (50) is an operating table.

## Revendications

1. Procédé (10) de commande sécurisée d'un appareil médical (50) par le biais d'une unité de saisie (52) en utilisant une unité de vérification (54), l'unité de saisie (52) communiquant avec l'unité de vérification (54) par le biais d'un canal de communication (56a, 56b), le procédé (10) comprenant les étapes:
- première détection (12), par l'unité de saisie (52), d'un premier actionnement (64a) au niveau d'un premier point d'actionnement (58) à l'intérieur d'une zone de validation (60) sur l'unité de saisie (52), le premier actionnement (64a) étant un toucher et une persistance du toucher,
- première émission (16), de l'unité de saisie (52) à l'unité de vérification (54), d'une demande (106) d'une information contextuelle (62) univoque, qui établit une association univoque entre l'actionnement sur l'unité de saisie (52) et l'unité de vérification (54),
- génération (18), par l'unité de vérification (54), de l'information contextuelle (62),
- deuxième émission (20), par l'unité de vérification (54) à l'unité de saisie (52), de l'information contextuelle (62),
- deuxième détection (22), par l'unité de saisie (52), d'un deuxième actionnement (64b), qui suit le premier actionnement lors d'un maintien persistant du toucher, le deuxième actionnement (64b) étant un mouvement du premier point d'actionnement (58) vers un deuxième point d'actionnement (66) à l'intérieur d'une zone de commande (68a) sur l'unité de saisie (52) avec un maintien persistant du toucher, et
- commande (24) de l'appareil médical (50) avec les étapes suivantes qui se répètent :
- troisième détection (26), par l'unité de saisie (52), d'un troisième actionnement (64c), le troisième actionnement étant un changement de place à l'intérieur de la zone de commande (68a) avec un maintien persistant du toucher,
- troisième émission (28), par l'unité de saisie (52) à l'unité de vérification (54), d'un message de commande (70), qui possède une instruction de commande (72), une information d'identification (74) et une information de changement de place (76), l'instruction de commande (72) étant associée à la zone de commande (68a), l'information d'identification (74) possédant l'information contextuelle (62) ou une information dérivée de l'information contextuelle (62) et l'information de changement de place (76) possédant des informations dans lesquelles sont incluses la longueur et la direction du changement de place ou qui peuvent être déterminées à partir de celles-ci,
- premier contrôle (30), par l'unité de vérification (54), si les informations de changement de place (76) d'au moins un sous-ensemble des troisièmes actionnements effectués indiquent que le changement de place sur l'unité de saisie (52) suit une forme prédéfinie,
- deuxième contrôle (32), par l'unité de vérification (54), si l'information d'identification (74) correspond à l'information contextuelle (62), et
- quatrième émission (34), par l'unité de vérification (54) à l'appareil médical (50), de l'instruction de commande (72) ou d'une instruction dérivée de l'instruction de commande (72), lorsque le premier contrôle (30) et le deuxième contrôle (32) ont eu une conclusion positive.

2. Procédé selon la revendication 1, les étapes suivantes respectives du procédé n'étant pas exécutées si le toucher est interrompu pendant la mise en oeuvre du procédé.

3. Procédé selon l'une des revendications précédentes, le procédé (10) étant poursuivi (14) avec la première détection (12) dans le cas où le toucher prend fin pendant la mise en oeuvre du procédé.

4. Procédé selon l'une des revendications précédentes, l'unité de vérification (54) possédant un processeur d'instructions (80) et un processeur de vérification (82) et fournissant, avec le processeur d'instructions (80), l'instruction de commande (72) ou l'instruction dérivée de l'instruction de commande (72) pour la quatrième émission (34) et validant la quatrième émission (34) avec le processeur de vérification (82).

5. Procédé selon l'une des revendications précédentes, l'information contextuelle (62) présentant une information d'appareil de l'unité de vérification (54).

6. Procédé selon l'une des revendications précédentes, l'information contextuelle (62) possédant au moins un élément choisi dans un groupe constitué par une information de temps, une information de session, une information de séquence et une information de début.

7. Procédé selon l'une des revendications précédentes, la forme prédéfinie étant une forme ovale, une forme elliptique ou une forme circulaire.

8. Procédé selon l'une des revendications précédentes, le canal de communication (56a, 56b) étant exactement un canal physique et/ou un canal logique, et en particulier l'instruction de commande (72) étant émise sous la forme d'exactement un message.

9. Procédé selon l'une des revendications précédentes, le procédé (10) étant poursuivi avec la première détection (12) dans le cas où l'information de changement de place (76) se répète de manière identique ou au sein d'une plage de tolérance prédéfinie.

10. Système (48) comprenant un appareil médical (50), une unité de saisie (52), au moins un processeur et au moins une mémoire (96a, 96c), l'au moins une mémoire (96a, 96c) contenant des instructions qui ont pour effet, lorsqu'elles sont exécutées par l'au moins un processeur, que l'au moins un processeur met en oeuvre un procédé (10) selon l'une des revendications précédentes.

11. Système selon la revendication 10, l'au moins un processeur possédant un processeur d'instructions (80) et un processeur de vérification (82), le processeur de vérification (82) étant configuré pour le premier et le deuxième contrôle (30, 32) ainsi qu'une validation (124) de l'instruction de commande (72) ou de l'instruction dérivée de l'instruction de commande (72) et le processeur d'instructions (80) étant configuré pour une fourniture de l'instruction de commande (72) ou de l'instruction dérivée de l'instruction de commande (72).

12. Système selon la revendication 10 ou 11, l'unité de saisie (52) possédant un écran tactile ou une unité de détection de geste, notamment en association avec des lunettes de réalité virtuelle.

13. Système selon l'une des revendications 10 à 12, l'unité de saisie (52) étant configurée pour une saisie au moyen de la main et/ou du doigt d'un utilisateur.

14. Système selon l'une des revendications 10 à 13, l'appareil médical (50) étant une table d'opération.
